# FASCICULE DE BREVET EUROPEEN

(11) **EP 1 129 694 B1**
(45) Date de publication et mention de la délivrance du brevet: **07.09.2005**
(21) Numéro de dépôt: 01400308.1
(22) Date de dépôt: 08.02.2001
(51) Int. Cl.: A61K 7/32, A61K 7/48

(54) **Utilisation de composés permettant de modifier les propriétés physico-chimiques de la peau et/ou des muqueuses en tant qu'agents empêchant ou diminuant l'adhésion des micro-organismes sur ces dernières**
Verwendung von Verbindungen zur Modifizierung der physico-chemischen Eigenschaften der Haut und/oder der Schleimhaut als Mittel zur Verhinderung oder Reduzierung der Adhäsion von Mikro-organismen darauf
Use of components for modifying the physico-chemical properties of the skin and/or the mucous membrane as agents for preventing or reducing the adhesion of micro-organisms thereon

(30) Priorité: 15.02.2000 FR 0001841
(43) Date de publication de la demande: 05.09.2001
(73) Titulaire: L'OREAL, 75008 Paris (FR)
(72) Inventeur: Cupferman, Sylvie, 94240 L'Hay Les Roses (FR); Lerebour, Géraldine, 91200 Athis-Mons (FR); Guillou, Véronique, 92160 Antony (FR); Simon, Pascal, 94400 Vitry-sur-Seine (FR)
(74) Mandataire: Casalonga, Axel

(56) Documents cités:
- EP-A- 0 315 912
- EP-A- 0 613 694
- EP-A- 0 697 213
- EP-A- 0 875 239
- WO-A-95/31956
- CH-A5- 688 787
- DE-A- 19 503 423
- DE-A- 19 634 021
- DE-A- 19 643 585
- DE-A1- 4 134 137
- DE-A1- 4 330 664
- DE-A1- 19 634 959
- FR-A1- 2 689 011
- FR-A1- 2 768 925
- US-A- 4 172 149
- US-A- 4 451 480
- YOUSEF, N. E. ET AL: "Inhibition of bacterial adherence and biofilm on contact lenses" EGYPT. J. BIOMED. SCI. (1998), 1, 79-94, 1998, XP002158695
- PATENT ABSTRACTS OF JAPAN vol. 017, no. 617 (C-1129) 15 Novembre 1993 & JP 05 186 328 A (KAO CORP) 27 Juillet 1993

## Description

L'invention concerne l'utilisation de composés permettant de modifier les propriétés physico-chimiques de la surface de la peau et/ou des muqueuses dans une composition cosmétique ou pour la préparation d'une composition pharmaceutique en tant qu'agents empêchant ou diminuant l'adhésion des micro-organismes, particulièrement des bactéries, sur la peau et/ou les muqueuses.

La peau humaine est peuplée en permanence d'une multitude de micro-organismes différents (bactéries, levures et champignons). La flore microbienne résidente, indispensable à la bonne santé de la peau, est constituée principalement de staphylocoques (*Staphylococcus epidermis* et *Staphylococcus hominis*), de corynebactéries, de propionibactéries Gram+ telles que *Propionibacterium acnes*, ainsi que d'une flore fongique principalement composée de *Pytosporum ovale.*

Les infections cutanées sont le plus souvent dues à la rupture de l'équilibre écologique de la flore résidente suite à la colonisation de la peau par des germes exogènes pathogènes ou à la prolifération anormale d'une souche endogène. Les germes pathogènes les plus connus sont *Pseudomonas aeruginosa* (Gram -) qui est responsable de petits boutons, de folliculites, de rougeurs et de prurit, *Candida albicans* pouvant provoquer des inflammations à la commissure des lèvres, des candidoses cutanées, du prurit, des folliculites et des aphtes, *Staphylococcus aureus* pouvant provoquer des boutons, des folliculites, de l'impétigo, et des furoncles, et *Streptococcus* du groupe A responsable d'impétigo.

Le document EP 315912 décrit l'utilisation de composés ayant une action anti-séborrhéique. Le document WO 95/31956 décrit une émulsion antibactérienne constituée d'une huile et d'un ester de glycérol afin d'inactiver la bactérie *Helicobacter pylori*. Le document DE 19634021 décrit l'utilisation d'un ou plusieurs composés choisis dans le groupe des glycoglycérolipides en tant que principes actifs anti-adhésifs contre les microorganismes, virus, parasites et protozoaires. Le document JP 05/186328 décrit une composition pour nettoyer la peau à base d'une huile végétale et d'un alcool en présence d'un polysiloxane volatil. Le document DE 4330664 décrit une composition contenant au moins une huile végétale pour traiter des maladies comme le psoriasis ou la dermatite. Le document FR 2768925 décrit une composition cosmétique comportant des extraits de lierre et de tournesol, utilisée contre les agressions chimiques de l'environnement sur la peau. Le document DE 19634959 décrit une composition pour son usage dans différentes maladies de la peau comprenant une huile de camomille, germe de blé ou d'amande douce. Le document US 4172149 décrit une composition cosmétique comprenant des triglycérides pour son utilisation dans les cas de sécrétion excessive de sébum. Le document DE 4134137 décrit une lotion bioactive pour stimuler la croissance des cheveux et éviter la formation de pellicules. Le document FR2689011 décrit une préparation cosmétique pour combattre les lésions cutanées labiales à base d'huile végétale, de teinture végétale et d'huiles essentielles. Le document US 4451480 décrit une composition pour le traitement de l'acné, comprenant des ozonides d'huiles. Le document CH 688787 décrit des compositions destinées aux soins de la peau comprenant des huiles naturelles.

Pour combattre ces micro-organismes, il est courant d'utiliser des antibiotiques ou bactéricides. L'utilisation de ces composés pose cependant le problème de la non spécificité d'action visant indifféremment la flore pathogène et la flore résidente, et le problème du risque d'apparition de résistances bactériennes, ainsi que des problèmes de tolérance cutanée (irritations, allergies).

Il est également connu de réduire ou de prévenir la colonisation de surfaces telles que les dents, la peau et/ou des muqueuses, par des germes pathogènes en empêchant leur fixation sur ces supports. Les composés utilisés en tant qu'agents anti-adhésion décrits dans l'art antérieur sont des hydrates de carbone et des dérivés d'hydrates de carbone (WO 96/23 479, EP 380 084, US 5 002 759, US 4 859 656, WO 81/03 175, WO 93/14 773, WO 95/15 149, WO 95/07 084 et WO 95/17 898).

Or, la plupart des hydrates de carbone constituent une source de carbone pour des bactéries et champignons. Leur présence dans des compositions cosmétiques favorise par conséquent la prolifération microbienne et nécessite l'augmentation de la concentration en agents conservateurs (bactéricides ou bactériostatiques). Cet inconvénient annule ainsi le bénéfice de l'approche consistant à remplacer des composés antibiotiques ou bactéricides par des composés réduisant l'adhérence microbienne.

La demanderesse a trouvé de manière surprenante qu'un groupe de composés particuliers, exempts de motifs hydrate de carbone, permettait de réduire significativement l'adhérence microbienne sur la peau et/ou les muqueuses et de prévenir ainsi la prolifération de germes potentiellement pathogènes en l'absence d'agents antibiotiques, bactéricides ou fongicides.

Ces composés, à la différence des hydrates de carbone qui se lient aux récepteurs des micro-organismes pour empêcher les liaisons aux glycolipides des cornéocytes, agissent sur les propriétés physico-chimiques de la surface de la peau et/ou des muqueuses, ces propriétés physico-chimiques faisant intervenir les interactions électrodynamiques dues au forces de Van der Waals, les interactions acido-basiques selon Lewis et les interactions électrostatiques.

En outre, ces composés ne sont pas bactéricides. De ce fait, ils ne provoquent pas d'effets secondaires indésirables sur la peau et/ou les muqueuses.

Les composés selon l'invention, utilisés en tant que principes actifs, permettent de réduire ou d'empêcher l'adhésion d'un micro-organisme dont la charge globale de surface est négative ou positive en augmentant la charge respectivement négative ou positive de la peau, de manière à entraîner une répulsion entre la peau et/ou les muqueuses et le micro-organisme.

Les composés selon l'invention, utilisés en tant que principes actifs, permettent en outre de réduire ou d'empêcher l'adhésion d'un micro-organisme, en limitant le plus possible les interactions du type Van der Waals entre la peau et/ou les muqueuses et le micro-organisme, en favorisant les interactions répulsives de type acide-base selon Lewis et en limitant les interactions attractives de type acide-base selon Lewis entre le micro-organisme et la peau et/ou les muqueuses.

Par empêcher ou réduire l'adhésion des micro-organismes, il faut entendre que le composé ou la composition le contenant peut être utilisé aussi bien à titre préventif, pour sa capacité à prévenir, totalement ou partiellement, l'adhésion des micro-organismes, qu'à titre curatif pour sa capacité à faciliter le détachement des micro-organismes.

Ces composés sont choisis de manière à ce que le logarithme décimal du nombre moyen de bactéries viables adhérant sur l'épiderme reconstruit, après un test de mise en contact dudit épiderme avec le composé testé pendant 2 heures à 37°C, soit inférieur d'au moins 0,3 à celui obtenu par un test réalisé avec de l'eau dans les mêmes conditions.

L'épiderme reconstruit utilisé dans le test indiqué ci-dessus est l'épiderme humain reconstitué, équivalent de la peau humaine, vendu par la société EPISKIN.

Ce test permet d'évaluer les modifications des propriétés physico-chimiques de surface de la peau et/ou des muqueuses, faisant intervenir les interactions électrodynamiques de Van der Waals, les interactions acido-basiques selon Lewis et les interactions électrostatiques.

L'invention a donc pour objet l'utilisation en tant que principe actif, dans une composition cosmétique ou pour la préparation d'une composition pharmaceutique, d'une quantité efficace d'au moins un composé exempt de motifs hydrates de carbone, modifiant les propriétés physico-chimiques de la surface de la peau et/ou des muqueuses, en vue d'empêcher ou réduire l'adhésion des micro-organismes sur la peau et/ou les muqueuses, choisi de manière à ce que le logarithme décimal du nombre moyen de bactéries viables adhérant sur l'épiderme reconstruit, après un test de mise en contact dudit épiderme avec le composé testé pendant 2 heures à 37°C, soit inférieur d'au moins 0,3 à celui obtenu par un test réalisé avec de l'eau dans les mêmes conditions.

De préférence, on utilisera les composés pour lesquels le logarithme décimal ci-dessus défini est inférieur de 0,5 et plus particulièrement inférieur de 1 à celui de l'eau dans les mêmes conditions.

Le protocole expérimental permettant de choisir ces composés sera défini ci-dessous.

Ces composés peuvent être de natures complètement différentes et peuvent être choisis, à titre d'exemple non limitatif, parmi des corps gras non solides à température ambiante, des polymères, des tensio-actifs et/ou leurs mélanges.

Les composés utilisés selon l'invention peuvent très bien être hydrophiles ou lipophiles.

On utilise plus préférentiellement en tant que principe actif dans une composition cosmétique ou pour la préparation d'une composition pharmaceutique, une quantité efficace de composés exempts de motifs hydrates de carbone modifiant les propriétés physico-chimiques de la surface de la peau et/ ou des muqueuses choisis parmi les tensioactifs tels que le cocoamphodiacétate disodique, le cocoate de glycéryle oxyéthyléné (7 OE) comme le produit vendu par la société COGNIS sous la dénomination Cetiol HE, l'hexadécénylsuccinate 18, le PPG-15 stéaryl éther; "les sels de mono sulfosuccinate de monoéthanol amide ricinoleique comme le produit vendu par la société Goldschmidt sous la dénomination REWODERM 51333, le triglycéride ricinoleique hydrogéné oxyéthyléné à 60 motifs d'oxyde d'éthylène comme le produit vendu par la société Nikko sous la dénomination NIKK OL HCO-60 ou comme le produit vendu par la société BASF sous la dénomination CREMOPHOR RH60", les polymères tels que les Poloxamers, qui sont des copolymères séquencés d'oxyde d'éthylène et d'oxyde de propylène, comme par exemple le produit vendu sous la dénomination Lutrol F68 par la société BASF et le Poloxamer 407 vendu sous la dénomination SYNPERONIC PE/F 127 par la société UNIQEMA; "le polyacrylamide/C13-14 Isoparaffin/Laureth-7 comme le produit vendu par la société SEPPIC sous la dénomination SEPIGEL 305". Les corps gras non solides à température ambiante (c'est à dire à une température allant d'environ 20 à 35°C) tels que l'huile de sésame, l'huile d'amande douce, l'huile d'amande d'abricot, l'huile de tournesol, le palmitate d'octoxyglycéryle (ou palmitate d'éthyl-2-hexyl glycéryl éther) comme le produit commercialisé sous la dénomination Mexanyl GP par la société Chimex, le béhénate d'octoxyglycéryle (ou béhénate d'éthyl-2-hexyl glycéryl éther), l'adipate de dioctyle, le tartrate de di-alcools C₁₂-C₁₃ ramifiés comme le produit vendu sous la dénomination Cosmacol ETI par la société Enichem.

Selon l'invention, on utilise le ou les composé ou la composition le ou les contenant en application topique sur la peau et/ou les muqueuses.

L'adhésion de micro-organismes à la peau et/ou aux muqueuses a des conséquences qui vont du simple désagrément (l'odeur, les petits boutons) aux maladies plus ou moins graves.

Un des aspects de l'invention est donc de proposer l'utilisation d'un composé exempt de motifs hydrates de carbone en tant que principe actif dans une composition cosmétique ou pour la préparation d'une composition pharmaceutique.

Particulièrement, l'invention a pour objet l'utilisation cosmétique en application topique d'au moins un composé en tant que principe actif dans une composition cosmétique destinée à diminuer les mauvaises odeurs corporelles.

La flore microbienne de la surface de la peau est responsable d'un grand nombre de désordres.

La quantité de composé utilisable selon l'invention dépend bien évidemment de l'effet recherché et doit être une quantité efficace pour empêcher partiellement ou totalement l'adhésion des micro-organismes ou pour faciliter le détachement des micro-organismes.

A titre d'exemple, la quantité de composé utilisable selon l'invention peut aller par exemple de 0,1 à 100 %, de préférence de 0,5 à 50% et plus particulièrement de 1 à 25% du poids total de la composition.

Par milieu cosmétiquement acceptable, on entend un milieu compatible avec la peau, le cuir chevelu, les muqueuses, les ongles, et les cheveux.

Les compositions cosmétiques et pharmaceutiques utilisées selon l'invention peuvent se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide ou d'une dispersion d'huile dans une phase aqueuse à l'aide de sphérules, ces sphérules pouvant être des nanoparticules polymériques telles que les nanosphères et les nanocapsules, ou mieux, des vésicules lipidiques de type ionique et/ou non-ionique.

Quand la composition selon l'invention comporte une phase grasse, celle-ci représente, de préférence, de 1 à 60% du poids total de la composition.

Cette phase grasse peut comporter une ou plusieurs huiles choisies de préférence dans le groupe constitué par :
- les silicones volatiles ou non-volatiles, linéaires, ramifiées ou cycliques, organo-modifiées ou non, hydrosolubles ou liposolubles,
- les huiles minérales telles que l'huile de paraffine et de vaseline,
- les huiles d'origine animale telles que le perhydrosqualène,
- les huiles d'origine végétale telles que l'huile d'amandes douces, l'huile d'avocat, l'huile de ricin, l'huile d'olive, l'huile de jojoba, l'huile de sésame, l'huile d'arachide, l'huile de macadamia, l'huile de pépins de raisin, l'huile de colza, l'huile de coprah,
- les huiles synthétiques telles que les isoparaffines,
- les huiles fluorées et perfluorées,
- les esters d'acides gras.

Elle peut aussi comporter comme matières grasses (un) ou plusieurs alcools gras, acides gras ou cires (paraffine, cire de polyéthylène, Carnauba, cire d'abeilles).

De façon connue, les compositions utilisées dans l'invention peuvent en outre contenir des adjuvants habituels dans le domaine cosmétique tels que des gélifiants et/ou épaississants classiques hydrophiles ou lipophiles; des actifs hydrophiles ou lipophiles; des conservateurs; des solvants; des antioxydants; des parfums; des émulsionnants; des agents hydratants; des agents pigmentants; des dépigmentants; des agents kératolytiques; des vitamines; des émollients; des séquestrants; des tensio-actifs; des polymères; des agents alcalinisants ou acidifiants; des charges; des agents anti-radicaux libres; des céramides; des filtres solaires (notamment ultra-violets); des répulsifs pour insectes; des agents amincissants; des matières colorantes; des anti-pelliculaires.

Les quantités de ces différents adjuvants sont celles classiquement utilisées dans les domaines considérés.

Bien entendu, l'homme de l'art veillera à choisir le ou les éventuels composés à ajouter à la composition selon l'invention, de manière telle que les propriétés avantageuses attachées intrinsèquement à la composition conforme à l'invention ne soient pas, ou substantiellement pas, altérées par l'addition envisagée.

Comme solvants, on peut citer les solvants organiques hydrophiles, et par exemple les mono-alcools inférieurs, linéaires ou ramifiés, ayant de 1 à 8 atomes de carbone comme l'éthanol, le propanol, le butanol, l'isopropanol, l'isobutanol; les polyéthylèneglycols ayant de 6 à 80 oxydes d'éthylène, les polyols tels que le propylèneglycol, l'isoprène glycol, le butylèneglycol, le glycérol; les mono- ou dialkyles d'isosorbide dont les groupements alkyles ont de 1 à 5 atomes de carbone comme le diméthyl isosorbide; les éthers de glycol comme le diéthylène glycol mono-méthyle ou mono-éthyléther et les éthers de propylène glycol comme le dipropylène glycol méthyléther.

Les solvants organiques peuvent représenter de 5 à 98% du poids total de la composition.

Les compositions utilisées dans la présente invention peuvent être plus ou moins fluides et avoir l'aspect d'une crème blanche ou colorée, d'une pommade, d'un lait, d'une lotion, d'un sérum, d'une pâte, d'une mousse ou d'un solide.

Elles peuvent éventuellement être appliquées sur la peau sous forme d'aérosol.

Elles peuvent également se présenter sous forme solide, et par exemple sous forme de stick.

Elles peuvent être utilisées comme produit de soin, comme produit de nettoyage pour la peau ou les cheveux, comme produit solaire, en tant que produit de maquillage comme les fonds de teint, les rouges à lèvres, les mascaras, les fards, et/ou comme simple produit déodorant.

Le test d'anti-adhésion répond au protocole ci-dessous :

Avant l'adhésion bactérienne, l'épiderme reconstruit est mis en contact pendant 2 heures avec 25 mg du produit à tester à 37°C. On y ajoute alors 1 ml de suspension bactérienne de *Staphylococcus aureus* à une concentration de 10⁷ germes/ml dans du Tryptone sel. Après incubation 24 heures à 37°C, la suspension bactérienne est vidée et cinq rinçages sont réalisés par 1 ml d'eau distillée stérile. L'épiderme détaché de son support est alors broyé à l'aide d'un robot dans 18 ml de Tryptone sel. On effectue une dilution décimale de cette suspension dans le Tryptone sel, on procède ensuite à un ensemencement de ml de la dilution dans 15 ml de la gélose Trypticase Soja et à l'incubation pendant 24 heures à 37°C. On dénombre ensuite les cellules adhérentes et viables.

Ce test d'anti-adhésion permet d'évaluer l'efficacité de molécules seules ou de produits finis.

Avant le test d'anti-adhésion, on met en oeuvre le test de viabilité suivant :

Un mélange bactéries/produit à tester, dans le même rapport que dans le test anti-adhésion, est mis en contact 24 heures à 37°C. Le test peut nécessiter une incubation sous agitation pour éviter la mort des bactéries par manque d'oxygène, en particulier en ce qui concerne les corps gras non solide à température ambiante. Le dénombrement des germes est réalisé par dilution décimale dans du Tryptone sel et ensemencement au râteau de 100 µl sur de la gélose Trypticase Soja. Le comptage des colonies s'effectue après 24 heures d'incubation à 37°C.

L'essai de viabilité réalisé préalablement au test d'antiadhésion permet d'écarter toute composante bactéricide des molécules ou des produits finis testés et de ne mettre en évidence que l'activité anti-adhésion.

Les exemples suivants présentent les résultats obtenus pour différents composés testés selon l'invention et une réalisation particulière de composition selon l'invention.

Ces exemples sont bien entendu donnés à titre illustratif et n'ont absolument pas de caractère limitatif.

### Exemples de composés utilisés selon l'invention :

Les résultats obtenus, pour les composés ici présentés, résultent de la mise en oeuvre du protocole ci-dessus détaillé.

Les chiffres présentés en face du composé correspondent à la diminution du logarithme décimal du nombre moyen de *Staphylococcus aureus* viable adhérent sur l'épiderme reconstruit après traitement par le composé dans les conditions définies par le test précédent par rapport au logarithme décimal du nombre moyen de *Staphylococcus aureus* viable adhérent sur l'épiderme reconstruit après traitement à l'eau dans les mêmes conditions.

| | |
|---|---|
| Cocoamphodiacétate disodique | 1,34 |
| Cocoate de glycéryle oxyéthyléné (7 OE) | 1,41 |
| Sel de mono sulfoccinate de mono ethanol amide ricinoléique (testé à 5% en matière active) | 3,84 |
| Triglyacide ricinoléique hydrogéné oxyéthyléné vendu sous la dénomination NIKKOL HCO-60 par la société NIKKO (testé à 10% en matière active) | 0,8 |
| Poloxamer 407 : copolymère d'oxyde d'éthylène, d'oxyde de propylène et d'oxyde d'éthylène (98 OE/67 OP/98 OE) (PM : 12000) vendu sous la dénomination : SYNPERONIC PE/F 127 par la société UNIQEMA | 0,81 |
| Polyacrylamide/C13-14 Isoparaffin/Laureth-7 (testé à 1% en matière active) | 1,43 |
| Tartrate de di-alcools C₁₂-C₁₃ ramifiés | 2,31 |
| Huile d'amande d'abricot | 0,81 |
| Adipate de dioctyle | 0,90 |

**Exemple de composition utilisée selon l'invention:**

| Emulsion E/H pour le soin du visage : | |
|---|---|
| Poly méthylcétyl diméthyl méthylsiloxane oxyéthyléné (ABIL EM 90 ® de GOLDSCHMIDT) | 3 % |
| Palmitate d'éthyl-2-hexyl glycéryl éther (MEXANYL GP® de CHIMEX) | 10 % |
| Tartrate de di-alcools C₁₂-C₁₃ ramifiés (COSMACOL ETI ® de ENICHEM) | 10 % |
| Cocoate de glycéryle oxyéthyléné (7 OE) (CETIOL HE ® de GOGNIS) | 3 % |
| Condensat d'oxyde d'éthylène, d'oxyde de propylène et d'oxyde d'éthylène (PM : 8350) (75 OE/30 OP/75 OE) (LUTROL F 68 ® de BASF) | 3% |
| Eau | QSP 100 |
| Anti-oxydant | qs |
| Parfum | qs |

Après traitement par la composition ci-dessus, dans les conditions définies par le test précédent, on observe une diminution du logarithme décimal du nombre moyen de *Staphylococcus aureus* viable adhérent sur l'épiderme reconstruit par rapport au logarithme décimal du nombre moyen de *Staphylococcus aureus* viable adhérent sur l'épiderme reconstruit après traitement à l'eau dans les mêmes conditions de 3,96.

## Revendications

1. Utilisation en tant que principe actif, dans une composition cosmétique ou pour la préparation d'une composition pharmaceutique, d'une quantité efficace d'au moins un composé exempt de motifs hydrates de carbone, modifiant les propriétés physico-chimiques de la surface de la peau et/ou des muqueuses, en vue d'empêcher ou de réduire l'adhésion de micro-organismes sur ces dernières, ledit composé étant choisi de manière à ce que le logarithme décimal du nombre moyen de bactéries viables adhérant sur l'épiderme reconstruit, après un test de mise en contact dudit épiderme avec le composé testé pendant 2 heures à 37°C, soit inférieur d'au moins 0,3 à celui obtenu par un test réalisé avec de l'eau dans les mêmes conditions.

2. Utilisation en tant que principe actif, dans une composition cosmétique ou pour la préparation d'une composition pharmaceutique, d'une quantité efficace d'au moins un composé selon la revendication 1, **caractérisée en ce que** le logarithme décimal du nombre moyen de bactéries viables adhérant sur l'épiderme reconstruit, après un test de mise en contact dudit épiderme avec le composé testé pendant 2 heures à 37°C, est inférieur d'au moins 0,5 à celui obtenu par un test réalisé avec de l'eau dans les mêmes conditions.

3. Utilisation en tant que principe actif, dans une composition cosmétique ou pour la préparation d'une composition pharmaceutique, d'une quantité efficace d'au moins un composé selon l'une quelconque des revendications 1 ou 2, **caractérisée en ce que** le logarithme décimal du nombre moyen de bactéries viables adhérant sur l'épiderme reconstruit, après un test de mise en contact dudit épiderme avec le composé testé pendant 2 heures à 37°C, est inférieur d'au moins 1 à celui obtenu par un test réalisé avec de l'eau dans les mêmes conditions.

4. Utilisation selon l'une quelconque des revendications 1 à 3, **caractérisée en ce que** le principe actif est choisi parmi les tensioactifs, les polymères et les corps gras non solides à température ambiante.

5. Utilisation selon l'une quelconque des revendications 1 à 4, **caractérisée en ce que** le principe actif est choisi parmi, le cocoamphodiacétate disodique, le cocoate de glycéryle oxyéthyléné (7 OE), les sels de monosulfosuccinate de monoéthanol amide ricinoléique, le triglycéride ricinoléique hydrogéné oxyéthyléné à 60 motifs d'éthylène les Poloxamers, le polyacrylamide /C13-14 Isoparaffin/laureth-7, l'hexadécénylsuccinate 18, l'huile de sésame, le palmitate d'octoxyglycéryle, le béhénate d'octoxyglycéryle, l'adipate de dioctyle, le PPG-15 stéaryl éther, l'huile d'amande d'abricot, le tartrate de di-alcools C₁₂-C₁₃ ramifiés et leurs mélanges.

6. Utilisation selon l'une quelconque des revendications 1 à 5, **caractérisée en ce que** le principe actif est présent en une quantité allant de 0,1% à 100% du poids total de la composition.

7. Utilisation selon la revendication 6, **caractérisée en ce que** le principe actif est présent en une quantité allant de 0,5% à 50% du poids total de la composition.

8. Utilisation selon l'une quelconque des revendications 6 ou 7, **caractérisée en ce que** le principe actif est présent en une quantité allant de 1% à 25% du poids total de la composition.

9. Utilisation selon l'une quelconque des revendications 1 à 8, **caractérisée en ce que** la composition se présente sous la forme d'une lotion, d'un gel aqueux, d'un sérum, d'une émulsion ou d'une dispersion de vésicules lipidiques.

10. Utilisation cosmétique en application topique d'au moins un composé défini selon l'une quelconque des revendications 1 à 5 en tant que principe actif dans une composition cosmétique destinée à diminuer les mauvaises odeurs corporelles.

## Patentansprüche

1. Verwendung einer wirksamen Menge mindestens einer Verbindung, die keine Kohlenhydrateinheiten aufweist und die die physikalisch-chemischen Eigenschaften der Oberfläche der Haut und/oder der Schleimhäute verändert, um das Anhaften von Mikroorganismen auf der Haut und/oder den Schleimhäuten zu verhindern oder zu vermindern, als Wirkstoff in einer kosmetischen Zusammensetzung oder für die Herstellung einer pharmazeutischen Zusammensetzung, wobei die Verbindung so ausgewählt ist, dass der dekadische Logarithmus der mittleren Anzahl von lebensfähigen Bakterien, die an rekonstruierter Epidermis anhaften, nach einem Test unter in Kontakt Bringen der Epidermis mit der zu testenden Verbindung während 2 Stunden bei 37 °C mindestens 0,3 unter dem dekadischen Logarithmus liegt, der in einem Test erhalten wird, welcher mit Wasser unter den gleichen Bedingungen durchgeführt wird.

2. Verwendung einer wirksamen Menge mindestens einer Verbindung gemäß Anspruch 1 als Wirkstoff in einer kosmetischen Zusammensetzung oder für die Herstellung einer pharmazeutischen Zusammensetzung, **dadurch gekennzeichnet, dass** der dekadische Logarithmus der mittleren Anzahl von lebensfähigen, an der rekonstruierten Epidermis anhaftenden Bakterien nach einem Test unter in Kontakt Bringen der Epidermis mit der zu testenden Verbindung während 2 Stunden bei 37 °C mindestens 0,5 unter dem Wert liegt, der in einem Test erhalten wird, der mit Wasser unter den gleichen Bedingungen durchgeführt wird.

3. Verwendung einer wirksamen Menge mindestens einer Verbindung nach einem der Ansprüche 1 oder 2 als Wirkstoff in einer kosmetischen Zusammensetzung oder für die Herstellung einer pharmazeutischen Zusammensetzung, **dadurch gekennzeichnet, dass** der dekadische Logarithmus der mittleren Anzahl von lebensfähigen, an der rekonstruierten Epidermis anhaftenden Bakterien nach einem Test unter in Kontakt Bringen der Epidermis mit der zu testenden Verbindung während 2 Stunden bei 37 °C mindestens 1 unter dem Wert liegt, der in einem Test erhalten wird, der mit Wasser unter den gleichen Bedingungen durchgeführt wird.

4. Verwendung nach einem der Ansprüche 1 bis 3, **dadurch gekennzeichnet, dass** der Wirkstoff unter den grenzflächenaktiven Stoffen, Polymeren und bei Umgebungstemperatur nicht festen Fettsubstanzen ausgewählt ist.

5. Verwendung nach einem der Ansprüche 1 bis 4, **dadurch gekennzeichnet, dass** der Wirkstoff ausgewählt ist unter Dinatriumcocoamphodiacetat, ethoxyliertem (7 EO) Glycerylcocoat, Monoethanolamidricinolsäure-monosulfosuccinatsalzen, mit 60 Einheiten Ethylenoxid ethoxyliertem, hydriertem Ricinolsäuretriglycerid, Poloxameren, Polyacrylamid/C13-14 Isoparaffin/Laureth-7, Hexadecenylsuccinat 18, Sesamöl, Octoxyglycerylpalmitat, Octoxyglycerylbehenat, Dioctyladipat, PPG-15-stearylether, Aprikosenkernöl, dem Tartrat von verzweigten C₁₂₋₁₃-Alkoholen und deren Gemischen.

6. Verwendung nach einem der Ansprüche 1 bis 5, **dadurch gekennzeichnet, dass** der Wirkstoff in einer Menge von 0,1 bis 100 % des Gesamtgewichts der Zusammensetzung vorliegt.

7. Verwendung nach Anspruch 6, **dadurch gekennzeichnet, dass** der Wirkstoff in einer Menge von 0,5 bis 50 % des Gesamtgewichts der Zusammensetzung vorliegt.

8. Verwendung nach einem der Ansprüche 6 oder 7, **dadurch gekennzeichnet, dass** der Wirkstoff in einer Menge von 1 bis 25 % des Gesamtgewichts der Zusammensetzung vorliegt.

9. Verwendung nach einem der Ansprüche 1 bis 8, **dadurch gekennzeichnet, dass** die Zusammensetzung als Lotion, wässeriges Gel, Serum, Emulsion oder Dispersion von Lipidvesikeln vorliegt.

10. Kosmetische Verwendung mindestens einer Verbindung nach einem der Ansprüche 1 bis 5 zur topischen Anwendung als Wirkstoff in einer kosmetischen Zusammensetzung, die dazu vorgesehen ist, schlechte Körpergerüche zu vermindern.

## Claims

1. Use, as active ingredient, in a cosmetic composition or for the preparation of a pharmaceutical composition, of an effective quantity of at least one compound free of carbohydrate units, modifying the physicochemical properties of the surface of the skin and/or of the mucous membranes, so as to prevent or reduce the adhesion of microorganisms to the skin and/or the mucous membranes, chosen so that the decimal logarithm of the mean number of viable bacteria adhering to reconstructed epidermis, after a test consisting in bringing the said epidermis into contact with the test compound for 2 hours at 37°C, is at least 0.3 less than that obtained by a test carried out with water under the same conditions.

2. Use, as active ingredient, in a cosmetic composition or for the preparation of a pharmaceutical composition, of an effective quantity of at least one compound according to Claim 1, **characterized in that** the decimal logarithm of the mean number of viable bacteria adhering to reconstructed epidermis, after a test consisting in bringing the said epidermis into contact with the test compound for 2 hours at 37°C, is at least 0.5 less than that obtained by a test carried out with water under the same conditions.

3. Use, as active ingredient, in a cosmetic composition or for the preparation of a pharmaceutical composition, of an effective quantity of at least one compound according to either of Claims 1 and 2, **characterized in that** the decimal logarithm of the mean number of viable bacteria adhering to reconstructed epidermis, after a test consisting in bringing the said epidermis into contact with the test compound for 2 hours at 37°C, is at least 1 less than that obtained by a test carried out with water under the same conditions.

4. Use according to any one of Claims 1 to 3, **characterized in that** the active ingredient is chosen from surfactants, polymers and nonsolid fatty substances at room temperature.

5. Use according to any one of Claims 1 to 4, **characterized in that** the active ingredient is chosen from disodium cocoamphodiacetate, oxyethylenated glyceryl cocoate (7 EO), the ricinoleic monoethanolamide monosulphosuccinate salts, oxyethylenated hydrogenated ricinoleic triglyceride containing 60 ethylene units, Poloxamers, polyacrylamide/C13-14 Isoparaffin/Laureth-7 hexadecenyl succinate 18, sesame oil, octoxyglyceryl palmitate, octoxyglyceryl behenate, dioctyl adipate, PEG-15 stearyl ether, apricot stone oil, tartrate of branched C₁₂-C₁₃ dialcohols and mixtures thereof.

6. Use according to any one of Claims 1 to 5, **characterized in that** the active ingredient is present in a quantity ranging from 0.1% to 100% of the total weight of the composition.

7. Use according to Claim 6, **characterized in that** the active ingredient is present in a quantity ranging from 0.5% to 50% of the total weight of the composition.

8. Use according to either of Claims 6 and 7, **characterized in that** the active ingredient is present in a quantity ranging from 1% to 25% of the total weight of the composition.

9. Use according to any one of Claims 1 to 8, **characterized in that** the composition is provided in the form of a lotion, an aqueous gel, a serum, an emulsion or a dispersion of lipid vesicles.

10. Cosmetic use by topical application of at least one compound as defined according to any one of Claims 1 to 5 as active ingredient in a cosmetic composition intended to reduce bad body odours.
